# EUROPEAN PATENT SPECIFICATION

(11) **EP 2 744 429 B1**
(45) Date of publication and mention of the grant of the patent: **30.03.2016**
(21) Application number: 12761879.1
(22) Date of filing: 10.08.2012
(51) Int. Cl.: A61B 17/54

(54) **PEDICURE MILLER**
FRÄSER FÜR PEDIKÜRE
MACHINE À FRAISER DE PÉDICURE

(30) Priority: 17.08.2011 CZ 20110507; 20.06.2012 CZ 20120411
(43) Date of publication of application: 25.06.2014
(73) Proprietor: Novak, Frantisek, 50002 Hradec Kralove (CZ)
(72) Inventor: Novak, Frantisek, 50002 Hradec Kralove (CZ)
(74) Representative: Kratochvil, Vaclav
(86) International application number: PCT/CZ2012/000079
(87) International publication number: WO 2013/023627

(56) References cited:
- DE-U1- 9 114 283
- DE-U1-202006 016 816
- US-A- 4 958 963
- US-A- 5 129 121

## Description

### Technical

The pedicure miller is designed for the easy, safe and fast removal of hard skin on the feet at home. It can be done after every bath or shower, and the gently removed skin is immediately and cleanly washed away.

### State of the art

Hard skin on the feet is currently removed at long intervals, mostly in the process of manual scrubbing at home. Electrical dry grinders are used exceptionally, as their disadvantage lies in the high rotations, which cause the dispersion of tiny skin particles in the air. Electrical drives are not suitable for use in bathrooms.
U.S. Patent document US 5,129,121 A discloses a turbine driven rotating brush including a turbine enclosed in a housing having an inlet for receiving water and an outlet for discharging water flow. This turbine driven rotatory brush further includes a port in the housing that directs water onto blades of the turbine, and a reduction gear that transmits the torque output of the turbine to a shaft on which a brush is mounted. This document is the closest prior art to the claimed invention.

Document DE 20 2006 016 816 U1 also discloses a turbine driven rotating brush turbine enclosed in a housing having an inlet for receiving water and an outlet for discharging water flow.

### Summary of the invention

The pedicure miller is connectable by a flexible hose to the water supply from the bathroom tap.
The pedicure miller comprises:
a box, in which there is a rotationally mounted turbine; a shaft fixed to the said turbine; and a tool having an external milling part,
wherein the box has its rear side firmly closed,
wherein the turbine comprises radial saucer-shaped blades enclosed at their back by a full side facing the rear side of the box, wherein the saucer-shaped blades are open at their front,
wherein a peripheral part of the body of the box comprises a water inlet connected to at least one jet, from which a water flow is directed substantially tangential to the rear sides of the saucer-shaped blades, and the water flowing over the said saucer-shaped blades is redirected to the open front sides of the saucer-shaped blades, where a water outlet is provided through a cap window at a front part of the box, and
wherein said shaft has the tool suitably fixed on its front section for the removal of hard skin on the feet.

The water inlet is distributed in multiple jets located evenly in the peripheral part of the box body, so that the water flowing from the multiple jets along the full side is directed to the rear parts of the saucer-shaped blades.

The jets consist of a jet cube, which has in the parting line the jets shaped in the rear cube and the front cube. The rear cube and the front cube are coupled fast in the parting line.
The jet cube is located in the box cavity at the water intake from the box holder, where it is fixed at the rear by at least one supporting strip clicked into the cavity of the rear side and the front part of the jet cube is fixed by the perimeter of the turbine cap.
The jets of the jet cube are connected to the water intake from the box holder and the jet cube is sealed in the box.

The turbine space in the box is sealed by the turbine cap with a cap window for water outlet from the blades of the turbine.
On the perimeter of the turbine cap there is a large spring and a small spring that determine the position of the turbine cap in the box.

The turbine is fixed to its shaft, which has the rear pin rotation mounted in the box bearing of the box. The front section of the shaft is rotation mounted in the cap bearing of the turbine cap, through which the shaft leads to the front space of the box.

The turbine consists of the full side and the rear wheel and the front wheel firmly coupled together.
The front wheel has on the outer blade ends peripheral walls that shape the blades into half-saucers, including the external edges of the blades.

The mutual position of the rear wheel and the front wheel is determined by the connecting pin.
The central hole of the turbine has pressed grooving of the shaft so that the end of the grooving at the rear pin rests on the installation of the central hole by the rear side of the rear wheel.

The front section of the shaft has a thread, on which the tool is mounted using a tubular body with milling protrusions in the front side.

In the front section of the shaft, using the tubular body the tool is mounted and fixed so that the front section of the shaft has shaft mounting, on which the fork of the tool is mounted, and the tool has in the tubular body a transverse locking groove, where there is a safety pin locking the tool axially in the shaft groove of the shaft. The front side of the tubular body is firmly coupled to a disk equipped with milling protrusions at the front.

In the front space of the box there is a box lid fixed with a central orifice for the tool and inter-rib spaces open to the outside.
The box lid is equipped with a collar engaged in the front edge of the box.
The box lid collar has a sealing groove around the edge.

The box lid collar has lid catches around the edge, clicked in the mounting grooves of the box.
On the sides of the lid catches the depth of the mounting groove in the circumferential direction within a short distance reaches zero, which means in the turned position of the box lid the box lid can be removed from the box.

The pedicure miller has a distribution system, where in the showering position the water is directed to the front space of the box, and through the shower cap to the outside. In the milling position of the distribution system the water is directed from at least one jet into the turbine, from which via the front box space and the shower cap the water is led outside.

The turbine is located in the turbine cylinder of the box.
The distribution system is equipped with a switch rotating around the main axis of the turbine. The cylindrical section of the switch on the outer side of the turbine cylinder is mounted so that in the showering position the switch covers the turbine channel, and water from at least one jet is directed to the front space of the box. In the milling position the cylindrical section of the switch is turned slightly outside the turbine channel, and water from at least one jet is directed to the turbine.
The front part of the box includes a shower cap, where there is space for the tool in the middle. The switch is fixed to the inner side of the shower cap, which is rotation mounted in the front part of the box.

The front edge of the box has a cabinet thread, through which the cap thread of the shower cap is led, whose movement on the screw switches between the showering and the milling positions.
In the milling position of the shower cap with the switch, the shower cap is adjacent to the box. In the tool space the tool is relatively shifted out towards the shower cap so that between the rear side of the disk and the cap bottom there is a gap for the water outlet. The front side of the disk with the milling protrusions is in the front milling position.
In the showering position of the shower cap with the switch, the shower cap is moved outwards in the box. In the tool space the tool towards the shower cap is relatively shifted in so that the rear side of the disk rests on the cap bottom. The front side of the disk with the milling protrusions is engaged in the tool space.

### Figures

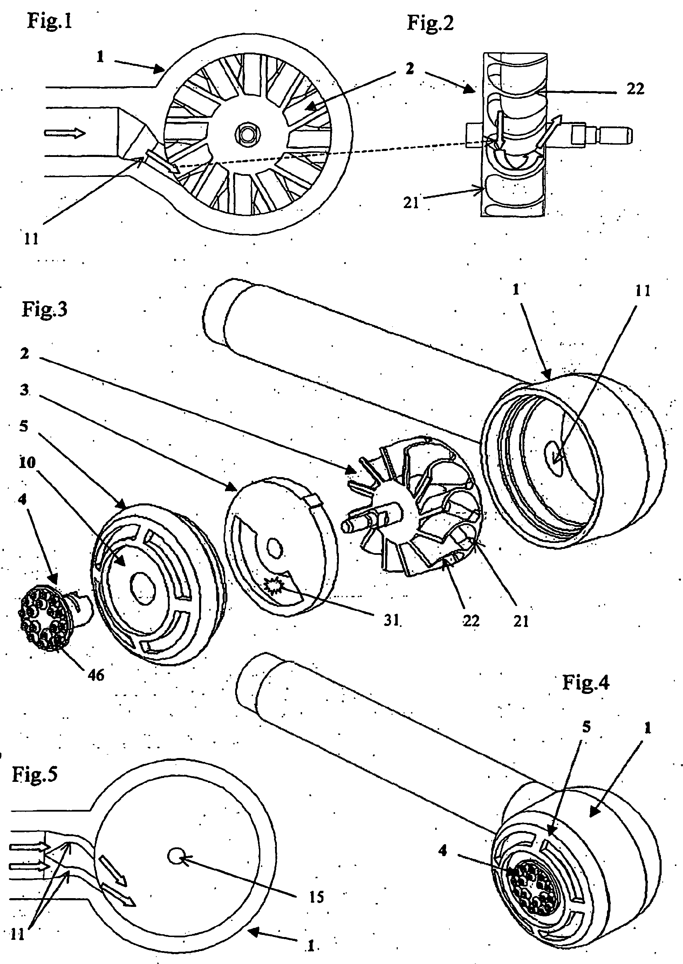

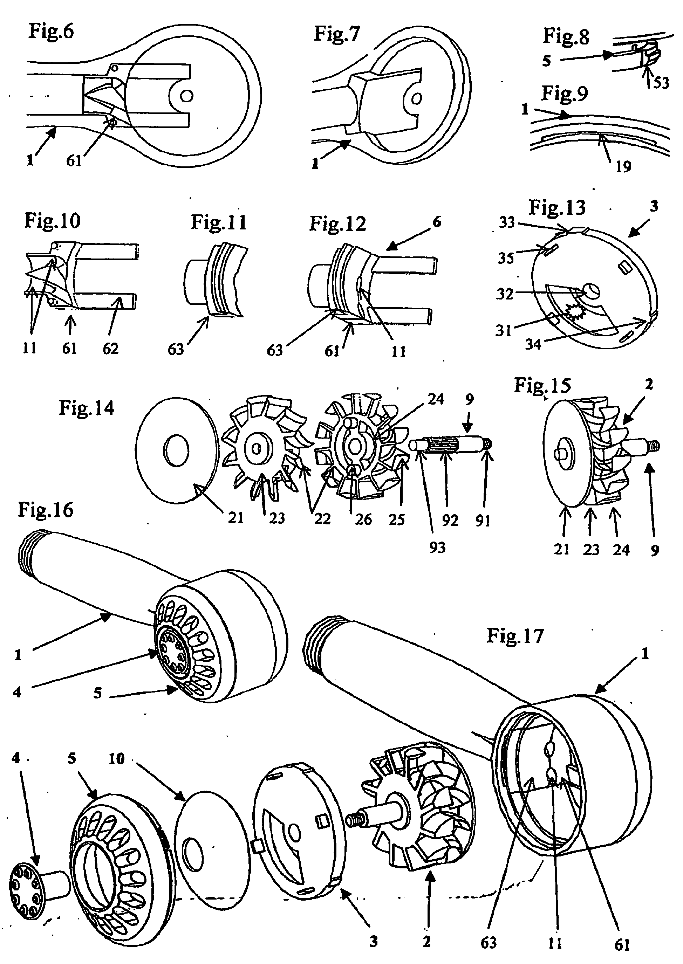

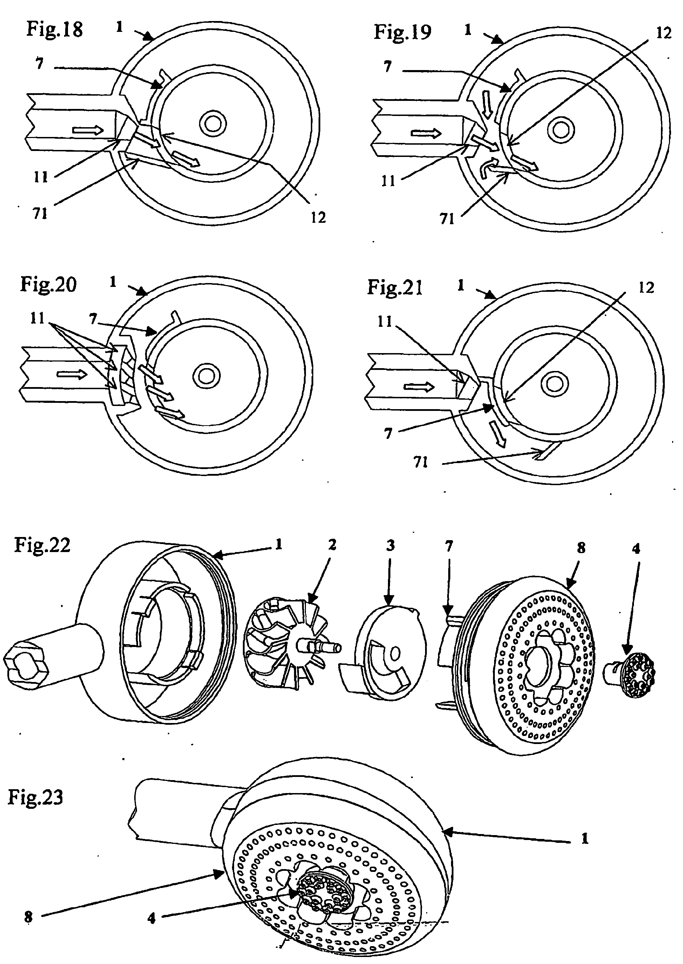

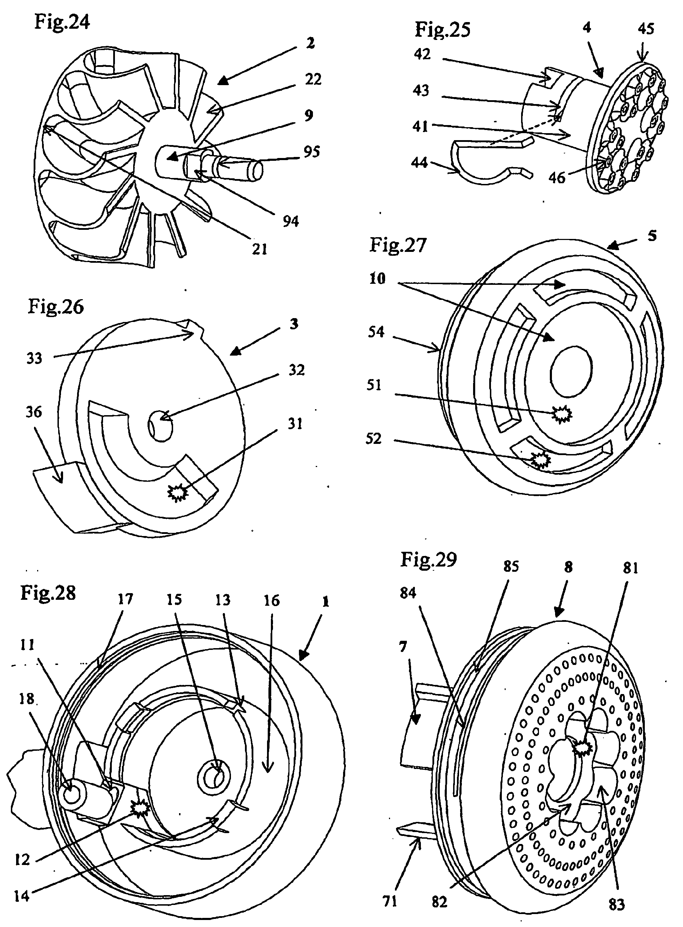

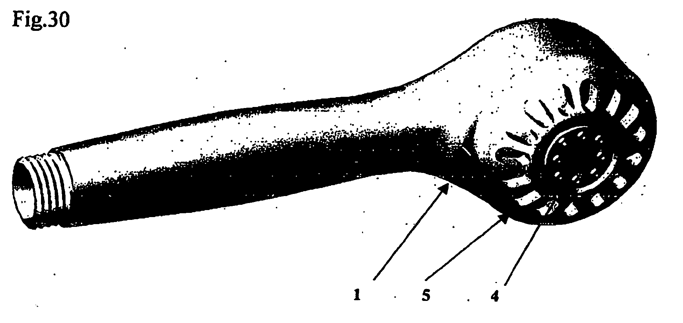

FIG. 1 shows the box and turbine section with arrows for the water flow; FIG. 2 shows the turbine with water flow arrows; FIG. 3 shows the disassembled miller set; FIG. 4 shows the assembled miller set; FIG. 5 shows the box and two jets section with arrows illustrating water flowing into the turbine; FIG. 6 shows a section of the box and parting line of the jet cube; FIG. 7 shows the section of the box with the cavity for the jet cube; FIG. 8 shows the lid catch; FIG. 9 shows the mounting groove of the box; FIG. 10 shows the rear jet cube; FIG. 11 shows the front jet cube; FIG. 12 shows the jet cube; FIG. 13 shows the turbine cap; FIG. 14 shows the disassembled turbine with the shaft; FIG. 15 shows the assembled turbine; FIG. 16 shows the assembled pedicure miller; FIG. 17 shows the disassembled pedicure miller; FIG. 18 shows the section of the shower box with the switch without re-circulation and arrows for water flowing into the turbine; FIG. 19 shows the section of the shower box with the switch with re-circulation and arrows for water flowing into the turbine; FIG. 20 shows the section of the box with three jets and re-circulation and arrows for water flowing into the turbine; FIG. 21 shows the section of the shower box with the switch in the position of water flowing into the shower; fog. 22 shows the disassembled shower set with the pedicure miller; FIG. 23 shows the assembled shower set with the pedicure miller; FIG. 24 shows the turbine; FIG. 25 shows the tool; FIG. 26 shows the turbine cap; FIG. 27 shows the lid of the separate pedicure miller; FIG. 28 shows the shower box with space for the turbine; FIG. 29 shows the shower cap with the switch and tool space; FIG. 30 is an actual view of the pedicure miller.

### Sample applications

The pedicure miller is connectable by a flexible hose to the water supply from the bathroom tap. The pedicure miller has a box where a turbine is rotation mounted, and the water intake in the box is directed to the turbine. The box **1** has a firmly closed rear side. The turbine **2** in the box **1** has sides made of saucer-shape blades **22** on the rear side closed with full side **21.** The sides of the saucer-shaped blades **22** at the front are open. In the peripheral part of the box **1** body the water inlet is distributed to at least one jet **11,** from which the water flow along the full side **21** is directed almost tangentially to the rear sides of the saucer-shaped blades **22.** The water flow is reversed on the saucer-shaped blades **22**, and led through the open sides of the blades **22,** where the water outlet aims through the cap window **31** to the front space of the box **1.** The turbine **2** with the shaft **9** has on its front section fixed the tool **4** with the external milling part for the removal of hard skin on the feet.

The water inlet can be distributed in two or more jets **11** located evenly in the peripheral part of the box body, so that the water flowing from the multiple jets **11** is by the full side **21** is directed to the rear parts of more saucer-shaped blades **22.**

The jets **11** consist of a jet cube **6** that has jet **11** shapes formed in the parting line in the rear cube **61** and the front cube **63.** The jet cube **6** is located in the box **1** cavity at the water intake from the box **1** holder, where it is fixed in the rear by at least one supporting strip **62** clicked into the cavity of the rear side of the box **1** and the front part of the jet cube **6** is fixed by the perimeter of the turbine cap **3**.

The jets **11** of the jet cube **6** are connected to the water intake from the box **1** holder and the jet cube **6** is sealed in the box **1.**
In the space of the water outlet from at least one jet **11** there can be at a minimum one re-circulation gap connected with the water space of the front part of the box, and water from at least one jet **11,** and the water from at least one re-circulation gap is along the full side **21** directed to the rear sides of the saucer-shaped blades **22.**

The turbine **2** space in the box **1** is sealed by the turbine cap **3** with the cap window **31** for water outlet from the blades **22** of the turbine **2**.
On the perimeter of the turbine cap **3** there is a large **33** spring and a small **34** spring that determine the position of the turbine cap **3** in the box **1.**
The large **33** and small **34** springs are located in the guiding grooves **13** of the box **1.** The turbine cap **3** is fixed to the box **1** either by the box lid **5** or box catches **14.**

The turbine **2** is fixed to its shaft **9,** which has the rear pin **93** rotation mounted in the box bearing **15** of the box **1.** The front section of the shaft **9** is rotation mounted in the turbine cap bearing **32,** through which the shaft leads to the front space of the box **1.**

The turbine **2** consists of the full side **21** and rear wheel **23** and front wheel **24** fixed fast together. The front wheel **24** has on the outer blade ends **22** peripheral sides **25** that shape the blades **22** into half-saucers, including the external edges of the blades **22.**

The mutual position of the rear wheel **23** and the front wheel **24** is determined by the connecting pin **26.** The central hole of the turbine **2** has pressed grooving **92** of the shaft **9** so that the end of the grooving **92** at the rear pin **93** rests on the installation of the central hole by the rear side of the rear wheel **23.**

The front section of the shaft **9** has a thread **91,** on which the tool **4** is mounted using the tubular body **41** with milling protrusions **46** in the front side.

In the front section of the shaft **9,** using the tubular body **41** the tool **4** is mounted and fixed so that the front section of the shaft **9** has shaft mounting **94,** on which the fork **42** of the tool **4** is mounted, and the tool **4** has in the tubular body **41** a transverse locking groove **43,** where there is a safety pin **44** locking the tool **4** axially in the shaft groove **95** of the shaft **9.**
The front side of the tubular body **41** is firmly coupled to the disk **45** equipped with milling protrusions **46** in the front.
The milling protrusion **46** can have a through-hole in the centre, connecting the front space of the box **1** through the disk **45** with the exterior. The milling protrusion **46** can have at least one edge on the loading side. The milling protrusions **46** can be made by punching holes in sheet metal. The disk **45** of the miller **4** can have holes connecting the front space of the box **1** through the disk **45** with the exterior.
The tool **4** can be a grinder with external grinding surface.

In the front space of the box **1** there is a box lid **5** fixed with a central orifice **51** for the tool **4** and inter-rib spaces **52** open to the outside.
The box lid **5** is equipped with a collar engaged in the front edge of the box **1.**
The box lid collar **5** has a sealing groove **54** around the edge. The edge of the sealing rests on the internal side of the peripheral casing of the box **1.**
The box lid collar **5** has lid catches **53** around the edge, clicked in the mounting grooves **19** of the box **1.** On the sides of the lid catches **53** the depth of the mounting groove **19** in the circumferential direction within a short distance reaches zero, which means in the turned position of the box lid **5** the box lid **5** can be removed from the box **1.**

There is at least one sieve **10** in the front space of the box **1** by the internal side of the box lid **5.** There are supporting strips **35** on the front side of the turbine cap **3**, between which at least one sieve **10** is placed, or the box lid **5** can have on its internal side at least one sieve **10.** The water outlet leads through the sieve **10** in the central **51** and inter-rib space **52.**

The flexible hose connect to the bathroom tap has one part of the quick coupler at the end to connect either the shower or the pedicure miller equipped with the quick coupler counterparts. The outer end of the holder has a thread, on which a quick coupler can be screwed to connect the pedicure miller to a flexible hose, on which there is the other part of the quick coupler. The holder is fixed to the peripheral casing of the box body **1,** whose input through-hole is connected to at least one jet **11.**

The pedicure miller is a component of a shower. The shower with the pedicure miller has a distribution system, where in the showering position the water is directed to the front space of the box **1,** and through the shower cap **8** to the outside. In the milling position of the distribution system the water is directed from at least one jet **11** into the turbine **2,** from which via the front box **1** space and the shower cap **8** the water is led outside.

The turbine **2** is located in the turbine cylinder **16** of the box **1.** The distribution system is equipped with a switch **7** rotating around the main axis of the turbine **2.** The cylindrical section of the switch **7** on the outer side of the turbine cylinder **16** is mounted so that in the showering position the switch **7** covers the turbine channel **12,** and water from at least one jet **11** is directed to the front space of the box **1.** In the milling position the cylindrical section of the switch **7** is turned slightly outside the turbine channel **12,** and water from at least one jet **11** is directed to the turbine **2**.

The front part of the box **1** includes the shower cap **8,** where there is space **81** for the tool in the middle. The switch **7** is fixed to the inner side of the shower cap **8** that is rotation mounted in the front part of the box **1.**

The front edge of the box **1** has a cabinet thread **17,** through which the cap thread **84** of the shower cap **8** is led, whose movement on the screw switches between the showering and the milling positions.
In the milling position of the shower cap **8** with the switch **7** the shower cap **8** is adjacent to the box **1.** In the tool space **81** the tool **4** is relatively shifted out towards the shower cap **8** so that between the rear side of the disk **45** and the cap bottom **82** there is a gap for the water outlet. The front side of the disk **45** with the milling protrusions **46** is in the front milling position. In the milling position of the shower cap **8** the water leaves through the tool space **81** and through the shower holes. To increase the water outlet space, the tool space **81** can be expanded with a cavity **83.**
In the showering position of the shower cap **8** with the switch **7** the shower cap **8** is moved outwards in the box **1.** In the tool space **81** the tool **4** is relatively shifted towards the shower cap **8** in so that the rear side of the disk **45** rests on the cap bottom **82.** The front side of the disk **45** with the milling protrusions **46** is engaged in the tool space **81.**
The shower cap **8** has a groove **85** for sealing purposes on the inner collar. The edge of the sealing rests on the internal side of the peripheral casing of the box **1.**
The switch **7** can be separated, controlled e.g. by a lever protruding from the box **1.**

In the milling position of the switch **7** without re-circulation, the switch **7** with partition **71** closes the gaps between the box body **1** with at least one jet **11,** and the external side of the turbine cylinder **16** by the turbine channel **12.**

In the milling position of the switch **7** with re-circulation of the water around the turbine channel **12** there is at least one re-circulation gap between the box body **1** with at least one jet **11**, and the external side of the turbine channel **12.** The re-circulation gap is connected to the water space of the front part of the box **1.** The switch **7** has a partition **71** through which circulating water is directed in the milling position from the large re-circulation gap to the turbine channel **12.**

The channel filling **36** is integrated into the turbine cap **3** so that the channel filling **36** fills the front part of the channel gap in the turbine cylinder **16.** The open rear part of the channel gap in the turbine cylinder **16** is the turbine channel **12.** The turbine cap **3** is fixed in the box **1** by pawls **14,** and the position of the turbine cap **3** is determined by the large **33** and small **34** spring in the guiding grooves **13** of the box **1.**

Another version of the distribution system can have a channel filling **36** that moves axially. In the engaged position of the channel filling **36** the turbine channel **12** is closed. In the disengaged position of the channel filling **36** the turbine channel **12** is open. The movable channel filling **36** is controlled by the lever protruding from the box **1.**

At the water outlet from at least one jet **11** there is the outlet of pumped air from the air channel 18. The air channel **18** is open in the showering position of the distribution system. The air channel **18** is closed in the milling position of the distribution system. The air channel **18** is opened and closed when switching between the showering and milling positions of the shower cap **8,** or by a separate lever protruding from the box **1.** By moving the shower cap **8** on the screw, the air intake into the air channel **18** is opened or closed. Air supply can have an additional control element to close the intake into the air channel **18,** or to regulate the air volume. The additional air creates a mixture of water and air for showering.

At the boundary between the hose and box **1** holder it is recommended to place a filtering sieve.

The pedicure miller can be made in three variants:
a) With a switch on the bathroom tap that either directs water into the shower hose or into the pedicure miller hose.
b) The flexible hose connect to the bathroom tap has one part of a quick coupler at the end to connect either the shower or the pedicure miller equipped with the quick coupler counterparts.
c) The hose of the shower combined with the pedicure miller is connected to the bathroom tap.

## Claims

1. A pedicure miller, connectable by a flexible hose to a bathroom tap,
wherein the pedicure miller comprises:
a box (1), in which there is a rotationally mounted turbine (2); a shaft (9) fixed to the said turbine (2); and
a tool (4) having an external milling part,
wherein the box (1) has its rear side firmly closed,
wherein the turbine (2) comprises radial saucer-shaped blades (22) enclosed at their back by a full side (21) facing the rear side of the box (1), wherein the saucer-shaped blades (22) are open at their front,
wherein a peripheral part of the body of the box (1) comprises a water inlet connected to at least one jet (11), from which a water flow is directed substantially tangential to the rear sides of the saucer-shaped blades (22), and the water flowing over the said saucer-shaped blades (22) is redirected to the open front sides of the saucer-shaped blades (22), where a water outlet is provided through a cap window (31) at a front part of the box (1), and
wherein said shaft (9) has the tool (4) suitably fixed on its front section for the removal of hard skin on the feet.

2. The pedicure miller according to claim 1 **characterized in that** the water inlet is distributed in multiple jets (11) located evenly in the peripheral part of the box body (1) so that the water flowing from the multiple jets (11) is by the full side (21) directed to the rear parts of the saucer-shaped blades (22).

3. The pedicure miller according to claim 2 **characterized in that** the jets (11) consist of the jet cube (6), which has in the parting line the jets (11) shaped in the rear cube (61) and the front cube (63), whereas the rear cube (61) and the front cube (63) are coupled fast in the parting line;
and that the jet cube (6) is located in the box (1) cavity at the water intake from the box holder (1), where it is fixed at the rear by at least one supporting strip (62) clicked into the cavity of the rear side (1) and the front part of the jet cube (6) is fixed by the perimeter of the turbine cap (3);
and that the jets (11) of the jet cube (6) are connected to the water intake from the box holder (1) and the jet cube (6) is sealed in the box (1).

4. The pedicure miller according to claim 1 **characterized in that** in the box (1) the turbine (2) space is sealed by the turbine cap (3), which has the cap window (31) for letting the water out from the blades (22) of the turbine (2);
and that on the perimeter of the turbine cap (3) there is a large spring (33) and a small spring (34) that determine the position of the turbine cap (3) in the box (1).

5. The pedicure miller according to claim 1 **characterized in that** the turbine (2) is fixed to its shaft (9) that has rear pin (93) rotation mounted in the box bearing (15) of the box (1), and the front part of the shaft (9) is rotation mounted in the cap bearing (32) of the turbine cap (3), where the shaft (9) passes through to the front section of the box (1).

6. The pedicure miller according to claim 5 **characterized in that** the turbine (2) consists of the full side (21) and the rear wheel (23) and the front wheel (24) firmly coupled together;
and that the front wheel (24) has on the outer blade ends (22) peripheral walls (25) that shape the blades (22) into half-saucers, including the external edges of the blades (22).

7. The pedicure miller according to claim 6 **characterized in that** the mutual position of the rear wheel (23) and the front wheel (24) is determined by the connecting pin (26);
and that the central hole of the turbine (2) has pressed grooving (92) of the shaft (9) so that the end of the grooving (92) at the rear pin (93) rests on the installation of the central hole by the rear side of the rear wheel (23).

8. The pedicure miller according to claim 1 **characterized in that** the front section of the shaft (9) has a thread (91), on which the tool (4) is mounted using the tubular body (41), with milling protrusions (46) in the front side.

9. The pedicure miller according to claim 1 **characterized in that** in the front section of the shaft (9), using the tubular body (41) there is a tool (4) mounted and fixed so that the front section of the shaft (9) has shaft mounting (94), on which the fork (42) of the tool (4) is mounted, and the tool (4) has in the tubular body (41) a transverse locking groove (43), where there is a safety pin (44) locking the tool (4) axially in the shaft groove (95) of the shaft (9);
and that the front side of the tubular body (41) is firmly coupled to the disk (45) equipped with the milling protrusions at the front (46).

10. The pedicure miller according to claim 1 **characterized in that** in the front space of the box (1) there is a box lid (5) fixed with a central orifice (51) for the tool (4) and the inter-rib spaces (52) open to the outside;
and that the box lid (5) is equipped with a collar engaged in the front edge of the box (1);
and that the box lid collar (5) has a sealing groove (54) around the edge;
and that the box lid collar (5) has lid catches (53) around the edge, clicked in the mounting grooves (19) of the box (1);
and that on the sides of the lid catches (53) the depth of the mounting groove (19) in the circumferential direction within a short distance reaches zero, which means in the turned position of the box lid (5) the box lid (5) can be removed from the box (1).

11. The pedicure miller according to claim 1 **characterized in that** least one sieve (10) is in the front space of the box (1) by the internal side of the box lid (5) and the water outlet leads through the sieve (10) in the central (51) and inter-rib space (52).

12. The pedicure miller according to claim 1 **characterized in that** the flexible hose connected to the bathroom tap has one part of the quick coupler at the end to connect either the shower or the pedicure miller equipped with the quick coupler counterparts,
and that the outer end of the holder of the pedicure miller has a thread, on which the quick coupler counterparts is screwed.

13. The pedicure miller according to claim 1 **characterized in that** the pedicure miller has a distribution system, where in the showering position the water is directed to the front space of the box (1), and through the shower cap (8) to the outside, and in the milling position of the distribution system the water is directed from at least one jet (11) into the turbine (2), from which via the front box space (1) and the shower cap (8) the water is led outside.

14. The pedicure miller according to claim 13 **characterized in that** the turbine (2) is located in the turbine cylinder (16) of the box (1);
and that the distribution system has a switch (7) that turns around the main axis of the turbine (2), and the cylindrical section of the switch (7) on the outer side of the turbine cylinder (16) is mounted so that in the showering position the switch (7) covers the turbine channel (12), and water from at least one jet (11) is directed to the front space of the box (1), and in the milling position the cylindrical section of the switch (7) is turned slightly outside the turbine channel (12), and water from at least one jet (11) is directed to the turbine (2);
and that the front part of the box (1) includes the shower cap (8), where there is space for the tool in the middle (81), and the switch (7) is fixed to the inner side of the shower cap (8) that is rotation mounted in the front part of the box (1).

15. The pedicure miller according to claim 14 **characterized in that** the front edge of the box (1) has a cabinet thread (17), through which the cap thread (84) of the shower cap (8) is led, whose movement on the screw switches between the showering and the milling positions;
and that in the milling position of the shower cap (8) with the switch (7) the shower cap (8) is adjacent to the box (1), and in the tool space (81) the tool (4) is relatively shifted out towards the shower cap (8) so that between the rear side of the disk (45) and the cap bottom (82) there is a gap for the water outlet, and the front side of the disk (45) with the milling protrusions (46) is in the front milling position;
and that in the showering position of the shower cap (8) with the switch (7) the shower cap (8) is shifted in the box (1) outward, and in the tool space (81) the tool (4) is relatively shifted towards the shower cap (8) in so that the rear side of the disk (45) rests on the cap bottom (82), and the front side of the disk (45) with the milling protrusions (46) is engaged in the tool space (81).

## Patentansprüche

1. Pedikärfräse zum Anschluss mit einem flexiblen Schlauch an die Wasserleitung,
wobei die Pedikärfräse aus folgenden Teilen besteht:
Gehäuse (1), in dem drehbar die Turbine (2) gelagert wird; Welle (9), angeschlossen zur Turbine (2); und
Werkzeug (4) mit dem äußeren Teil der Fräse,
wobei die Rückseite des Gehäuses (1) fest verschlossen ist,
wobei die Turbine (2) die Seitenbereiche der schalenförmigen Schaufeln (22) an der Rückseite durch die massive Wand (21) geschlossen hat und die Seitenbereiche der schalenförmigen Schaufeln (22) an der Frontseite offen sind,
wobei im Randbereich des Gehäusekörpers (1) der Wassereintritt in mindestens eine Düse (11) geführt wird, von der der Wasserstrom an der massiven Wand (21) nahezu tangential an die hinteren Teile der schalenförmigen Schaufeln (22) gerichtet ist und über die schalenförmigen Schaufeln (22) der Wasserstrom gewendet wird und durch die vorderen offenen Seitenteile der schalenförmigen Schaufeln (22) ausgeführt, wo sich der Wasseraustritt in der Deckelöffnung (31) in dem vorderen Bereich des Gehäuses (1) befindet, und
wobei die Welle (9) an dem vorderen Teil das Werkzeug (4) zur Beseitigung der harten Haut vom Fuß befestigt hat.

2. Pedikärfräse nach Anspruch 1, **dadurch gekennzeichnet, dass** der Wassereintritt in mehrere Düsen (11) verteilt wird, die kontinuierlich im Randbereich des Schrankgehäuses (1) so positioniert sind, dass die Wasserströme aus mehreren Düsen (11) an der massiven Wand (21) an die Brückenteile der mehr schalenförmigen Schaufeln (22) gerichtet sind.

3. Pedikärfräse nach Anspruch 2, **dadurch gekennzeichnet, dass** die Düsen (11) mit einem Düsenwürfel (6) gebildet sind, der in der Trennebene die Düsenformen (11) hat, geformt im hinteren Würfel (61) und vorderem Würfel (63), wobei der hintere Würfel (61) und der vordere Würfel (63) in der Trennebene fest verbunden sind,
und dadurch, dass der Düsenwürfel (6) sich in der Aussparung des Gehäuses (1) an der Stelle der Wasserzuleitung aus dem Griff des Gehäuses (1) befindet, wo er im Rückenteil mit mindestens einem Stützband (62) gesichert ist, das in der Aussparung der hinteren Gehäusefront (1) arretiert ist und der vordere Teil des Düsenwürfels (6) mit der Kontur des Turbinendeckels (3) gesichert wird,
und dadurch, dass die Düsen (11) des Düsenwürfels (6) an den Wasseranschluss vom Griff des Gehäuses (1) angeschlossen und der Düsenwürfel (6) im Schrank (1) abgedichtet ist.

4. Pedikärfräse nach Anspruch 1, **gekennzeichnet dadurch, dass** im Gehäuse (1) der Raum der Turbine (2) mit einem Turbinendeckel (3) geschlossen ist, in dem sich eine Öffnung (31) für Wasseraustritt aus den Schaufeln (22) der Turbine (2) befindet,
und dadurch, dass sich am Rande des Turbinendeckels (3) eine große Feder (33) und kleine Feder (34) befinden, mit denen die Position des Turbinendeckels (3) im Schrank (1) bestimmt ist.

5. Pedikärfräse nach Anspruch 1, **gekennzeichnet dadurch, dass** die Turbine (2) fest mit ihrer Welle (9) verbunden ist, die ihren hinteren Bolzen (93) drehbar im Gehäuselager (15) im Lager (1) gelagert hat und der vordere Teil der Welle (9) sich drehbar im Deckellager (32) des Turbinendeckels (3) befindet, durch den die Welle (9) in den frontalen Bereich des Gehäuses (1) verläuft.

6. Pedikärfräse nach Anspruch 5, **gekennzeichnet dadurch, dass** die Turbine (2) aus einer massiven Wand (21), des hinteren Rades (23) und vorderen Rades (24) besteht, die miteinander fest verbunden sind,
und dadurch, dass das Vorderrad (24) an den äußeren Enden der Schaufel (22) Außenwände (25) versehen ist, mit denen die Schaufeln (22) mit Halbschalenformen einschließlich der äußeren Beendigung der Schaufeln (22) geformt sind.

7. Pedikärfräse nach Anspruch 6, **gekennzeichnet dadurch, dass** die gegenseitige Position des hinteren Rads (23) und des vorderen Rads (24) mit dem Verbindungsbolzen (26) gegeben ist,
und dadurch, dass in das mittlere Loch der Turbine (2) die Kerbverzahnung (92) der Welle (9) so eingepresst ist, dass das Ende der Kerbverzahnung (92) am hinteren Bolzen (93) an den Ansatz des mittleren Lochs an der Rückseite des hinteren Rades (23) angelehnt ist.

8. Pedikärfräse nach Anspruch 1, **gekennzeichnet dadurch, dass** der vordere Teil der Welle (9) mit Gewinde (91) versehen ist, auf dem mit einem Rohrkörper (41) Werkzeug (4) angeschraubt ist, an dessen vorderen Frontseite mit Fräsvorsätzen (46) versehen.

9. Pedikärfräse nach Anspruch 1, **gekennzeichnet dadurch, dass** am vorderen Teil der Welle (9) mit einem Rohrkörper (41) das Werkzeug (4) aufgeschoben und so befestigt ist, dass der vordere Teil der Welle (9) mit Wellenansatz (94) versehen ist, auf dem die Gabel (42) des Werkzeugs (4) aufgeschoben ist und das Werkzeug (4) in dem Rohrkörper (41) zur Absicherung eine querführende Nut (43) besitzt, in der sich eine Drahtsicherung (44) befindet, mit der das Werkzeug (4) axial in der Nut (95) der Welle (9) gesichert wird,
und dadurch, dass mit der vorderen Front des Rohrkörpers (41) eine Scheibe (45) fest verbunden ist, die an der vorderen Frontseite mit Fräsvorsätzen (46) versehen ist.

10. Pedikärfräse nach Anspruch 2, **gekennzeichnet dadurch, dass** zum vorderen Bereich des Gehäuses (1) der Gehäusedeckel (5) befestigt ist, dessen Mittenbereich (51) für das Werkzeug (4) und die Räume zwischen Rippen (52) in die unmittelbare Umgebung offen sind,
und dadurch, dass der Gehäusedeckel (5) einen Ringansatz hat, der in den vorderen Rand des Gehäuses (1) eingeschoben ist,
und dadurch, dass der Ringansatz des Gehäusedeckels (5) an seinem Umfang mit einer Dichtungsrille (54) für Dichtung versehen ist,
und dadurch, dass der Ringansatz des Gehäusedeckels (5) auf seinem Rand mit Deckelklinken (53) versehen ist, die in den Montagerinnen (19) des Gehäuses (1) eingerastet sind,
und dadurch, dass an den Seiten der Deckelklinke (53) die Tiefe der Montagerille (19) in der Konturrichtung auf einem kurzen Abschnitt zur Nulltiefe ausläuft, wodurch der Gehäusedeckel (5) in leicht verdrehter Position aus dem Gehäuse (1) herausgenommen werden kann.

11. Pedikärfräse nach Anspruch 1, **gekennzeichnet dadurch, dass** sich im vorderen Bereich des Gehäuses (1) an der Innenseite des Gehäusedeckels (5) mindestens ein Sieb (10) befindet und über das Sieb (10) erfolgt der Wasseraustritt durch den mittleren Raum (51) und Räume zwischen Rippen (52).

12. Pedikärfräse nach Anspruch 1, **dadurch gekennzeichnet, dass** der an die Mischbatterie angeschlossene Schlauch am Ende einen Teil der Schnellkupplung hat, an den entweder Dusche oder die Pedikärfräse angeschlossen werden, die mit Gegenteilen der Schnellkupplung versehen sind,
und dadurch, dass das äußere Ende des Gerichts der Pedikärfräse mit einem Gewinde versehen ist, auf dem das Gegenstück der Schnellkupplung aufgeschraubt ist.

13. Pedikärfräse nach Anspruch 1, **dadurch gekennzeichnet, dass** die Dusche mit der Pedikärfräse mit einem Verteilsystem versehen ist, in dessen Duschposition das Wasser in den vorderen Bereich des Gehäuses (1) und durch den Duschdeckel (8) in die benachbarte Umgebung und in der Fräsposition des Verteilsystems das Wasser mindestens aus einer Düse (11) in die Turbine (2) strömt, von der das Wasser über den vorderen Bereich des Gehäuses (1) und den Duschdeckel (8) in die benachbarte Umgebung austritt.

14. Pedikärfräse nach Anspruch 13, **dadurch gekennzeichnet, dass** Turbine (2) sich im Turbinenzylinder (16) des Gehäuses (1) befindet,
und dadurch, dass das Verteilsystem mit einem Umschalter (7) versehen ist, der sich drehbar um die Hauptachse der Turbine (2) befindet und der zylindrische Ausschnitt des Umschalters (7) an der Außenseite des Turbinenzylinders (16) so installiert ist, dass in der Duschposition mit dem Umschalter (7) der Turbinenkanal (12) überdeckt ist und das Wasser zumindest aus einer Düse (11) in den vorderen Bereich des Gehäuses (1) gerichtet ist und in der Fräsposition der zylindrische Ausschnitt des Umschalters (7) außerhalb des Turbinenkanals (12) gedreht wurde und das Wasser von mindestens einer Düse (11) in die Turbine (2) gerichtet ist,
und dadurch, dass im vorderen Teil des Gehäuses (1) der Duschdeckel eingelegt wurde (8), in dessen Mitte sich der Werkzeugbereich befindet (81) und der Umschalter (7) ist zu der Innenseite des Duschdeckels (8) befestigt, der sich drehbar im vorderen Teil des Gehäuses (1) befindet.

15. Pedikärfräse nach Anspruch 14, **dadurch gekennzeichnet, dass** der vordere Rand des Gehäuses (1) mit dem Gehäusegewinde (17) versehen ist, durch das das Deckelgewinde (84) des Duschdeckels (8) geführt wird, dessen Schraubenbewegung zwischen der Dusch- und Fräsposition verläuft,
und dadurch, dass in der Fräsposition des Duschdeckels (8) mit Umschalter (7) der Duschdeckel (8) zum Gehäuse (1) zugeschoben wird und im Werkzeugbereich (81) befindet sich das Werkzeug (4) gegenüber dem Duschdeckel (8) so relativ ausgeschoben, dass zwischen der hinteren Seite der Scheibe (45) und dem Deckelboden (82) sich der Spalt für Wasseraustritt und die vordere Front der Scheibe (45) mit Fräsansätzen (46) sich in der vorderen Fräsposition befinden,
und dadurch, dass in der Duschposition des Duschdeckels (8) mit Umschalter (7) der Duschdeckel (8) im Gehäuse (1) in Richtung nach außen ausgeschoben wird und im Werkzeugbereich (81) befindet sich das Werkzeug (4) gegenüber dem Duschdeckel (8) so relativ eingeschoben, dass die Rückseite der Scheibe (45) an dem Deckelboden (82) angelehnt und die vordere Frontseite der Scheibe (45) mit Fräsansätzen (46) im Werkzeugbereich eingeschoben ist (81).

## Revendications

1. La machine à fraiser de pédicure raccordable par l'intermédiaire d'un tuyau flexible au réseau d'eau,
**caractérisée en ce que** la machine à fraiser de pédicure comprend :
Un boîtier (1) doté d'une turbine (2) montée à rotation; un arbre (9) fixé à la turbine (2) ; et un outil (4) doté d'une partie de fraisage externe,
le boîtier (1) étant bien fermée à l'arrière,
la turbine (2) dotée de pales coniques (22) fermées à l'arrière par une paroi pleine (21) et de pales coniques (22) ouvertes à l'avant,
l'entrée d'eau dans la partie circonférentielle du corps de boîtier (1) étant raccordée au moins a une buse (11) provoquant un courant d'eau près de la paroi pleine (21) orientée presque perpendiculairement vers l'arrière des pales coniques (22) et à travers des pales coniques (22) le courant d'eau est tourné et sorti par les côtés latéraux ouverts des pales coniques (22) où la sortie de l'eau est effectuée par une fenêtre de capot (31) vers la partie frontale du boîtier (1), et
l'arbre (9) doté de l'outil (4) dans la partie frontale (4) servant à enlever la peau dure du pied.

2. La machine à fraiser de pédicure selon la revendication 1, **caractérisée en ce que** l'eau d'entrée est distribuée vers des buses (11) multiples placés progressivement dans la partie périphérique du corps de boîtier (1) de façon que l'eau coulant à partir de buses (11) multiples soit orientée, près de la paroi pleine (21), vers la partie arrière des pales coniques (22).

3. La machine à fraiser de pédicure selon la revendication 2, **caractérisée en ce que** les buses (11) sont constituées par un cube de buses (6) qui présente, dans le plan de séparation, des buses (11) en forme de cube arrière (61) et de cube avant (63), le cube arrière (61) et le cube avant (63) étant fermement reliés dans le plan de séparation;
et **en ce que** le cube de buses (6) est situé dans le creux du boîtier (1) à l'endroit où l'eau arrive par la poignée du boîtier (1) où il est maintenu par au moins une bande de fixation (62) se fermant dans le creux de l'arrière du boîtier (1) et la partie avant du cube de buses (6) est maintenue par le capot de turbine (3); et **en ce que** les buses (11) du cube de buses (6) sont raccordées à l'eau arrivant par la poignée du boîtier (1) et le cube de buses (6) est scellé dans le boîtier (1).

4. La machine à fraiser de pédicure selon la revendication 1, **caractérisée en ce que** dans le boîtier (1) l'espace de la turbine est fermé par le capot de turbine (3) doté de la fenêtre de capot (31) pour laisser sortir l'eau des pales (22) de turbine (2),
et **en ce que** sur la circonférence du capot de turbine (3) il y a un grand ressort (33) et un petit ressort (34) qui déterminent la position du capot de turbine (3) dans le boîtier (1).

5. La machine à fraiser de pédicure selon la revendication 1, **caractérisée en ce que** la turbine (2) est fixée sur son arbre (9) dont l'axe arrière (93) est monté à rotation dans le palier de boîte (15) du boîtier (1) et la partie avant de l'arbre (9) est montée à rotation dans le palier de capot (32) du capot de turbine à travers duquel l'arbre (9) passe dans la partie avant du boîtier (1).

6. La machine à fraiser de pédicure selon la revendication 5, **caractérisée en ce que** la turbine (2) comprend une paroi pleine (21), une roue arrière (23) et une roue avant (24) qui sont fermement reliées ensemble,
et **en ce que** la roue avant (24) présente, sur les extrémités extérieures des pales (22), des parois périphériques (25) qui forment les pales (22) demi-coniques, y compris les bouts extérieurs des pales (22).

7. La machine à fraiser de pédicure selon la revendication 6, **caractérisée en ce que** la position mutuelle de la roue arrière (23) et de la roue avant (24) est déterminée par l'axe de connexion (26),
et **en ce que** les rainures (92) de l'arbre (9) sont disposées dans le trou central de la turbine (2) de façon que le bout des rainures (92) près de l'axe arrière (93) s'appuie sur l'épaulement du trou central près de la partie arrière de la roue arrière (23).

8. La machine à fraiser de pédicure selon la revendication 1, **caractérisée en ce que** la partie avant de l'arbre (9) est dotée d'un filetage (91) sur lequel l'outil (4) est vissé à l'aide d'un corps tubulaire (41), avec des saillies de fraisage (46) sur la face frontale.

9. La machine à fraiser de pédicure selon la revendication 1, **caractérisée en ce que** l'outil (4) est monté sur la partie avant de l'arbre (9) à l'aide du corps tubulaire (41) et fixé de façon que la partie avant de l'arbre (9) soit un montage à arbre (94) sur laquelle une fourche (42) de l'outil (4) est montée et le corps tubulaire (41) de l'outil (4) est doté d'une rainure transversale de verrouillage (43) où il y a un élément de sécurité (44) verrouillant axialement l'outil (4) dans la rainure d'arbre (95) de l'arbre (9),
et **en ce que** la face frontale du corps tubulaire (41) est fermement reliée au disque (45) qui est doté de saillies de fraisage (46) sur sa face frontale.

10. La machine à fraiser de pédicure selon la revendication 2, **caractérisée en ce que** sur la partie frontale du boîtier (1) un capot de boîtier (5) est fixé, avec un espace central (51) pour l'outil (4) et des espaces inter-nervures (52) ouvertes vers l'extérieur,
et **en ce que** le capot de boîtier (5) est doté d'un collet engagé dans le bord frontal du boîtier (1),
et **en ce que** le collet du capot de boîtier (5) est doté sur sa circonférence d'une rainure d'étanchéité (54) pour un joint,
et **en ce que** le collet du capot de boîtier (5) est doté sur sa circonférence de serrures de capot (53) s'enclenchant dans les rainures de montage (19) du boîtier (1),
et **en ce que** sur les côtés de la serrure de capot (53) la profondeur de la rainure de montage (19) dans la direction circonférentielle sur une courte distance atteint zéro, ce qui permet, dans la position tournée du capot de boîtier (5), d'enlever le capot de boîtier (5) du boîtier (1).

11. La machine à fraiser de pédicure selon la revendication 1, **caractérisée en ce que** dans la partie frontale du boîtier (1), près de l'espace intérieur du capot de boîtier (5) il y a au moins un tamis (10) qui laisse sortir l'eau qui passe par l'espace central (51) et par les espaces inter-nervures (52).

12. La machine à fraiser de pédicure selon la revendication 1, **caractérisée en ce qu'**une extrémité du tuyau flexible raccordé au mitigeur est munie d'un raccord rapide auquel est raccordée une douche ou une machine à fraiser de pédicure munies de contreparties du raccord rapide,
et **en ce que** l'extrémité extérieure de la poignée de la machine à fraiser de pédicure est dotée d'un filetage sur lequel la contrepartie du raccord rapide est vissée.

13. La machine à fraiser de pédicure selon la revendication 1, **caractérisée en ce que** la machine à fraiser de pédicure dispose d'un système de distribution, où, dans la position de douche, l'eau est dirigée vers la partie frontale du boîtier (1), et à travers le capot de douche (8) vers l'extérieur et, dans la position de fraisage du système de distribution, l'eau est dirigée à partir d'au moins une buse (11) vers la turbine (2), à partir de laquelle l'eau est dirigée à travers la partie frontale du boîtier (1) et le capot de douche (8) vers l'extérieur.

14. La machine à fraiser de pédicure selon la revendication 13, **caractérisée en ce que** la turbine (2) se trouve dans le cylindre de turbine (16) du boîtier (1),
et **en ce que** le système de distribution est doté d'un commutateur (7) qui tourne autour de l'axe principal de la turbine (2) et la section cylindrique du commutateur (7) se trouve à l'extérieur du cylindre de turbine (16) de façon que, dans la position de douche, le commutateur (7) recouvre le conduit de turbine (12) et l'eau d'au moins d'une buse (11) est dirigée vers la partie frontale du boîtier (1) et, dans la position de fraisage, la section cylindrique du commutateur (7) est tournée vers l'extérieur du conduit de turbine (12) et l'eau d'au moins d'une buse (11) est dirigée vers la turbine (2),
et **en ce que** dans la partie frontale du boîtier (1) il y a un capot de douche (8) et dans son centre, il y a un espace d'outil (81) et le commutateur (7) est fixé à l'intérieur du capot de douche (8) qui est monté à rotation dans la partie frontale du boîtier (1).

15. La machine à fraiser de pédicure selon la revendication 14, **caractérisée en ce que** le bord frontal du boîtier (1) comporte un filetage du boîtier (1) par lequel passe le filetage du capot (84) du capot de douche (8) dont le mouvement hélicoïdal permet de commuter entre la position de douche et la position de fraisage,
et **en ce que** dans la position de fraisage du capot de douche (8) avec le commutateur (7), le capot de douche (8) est placé près du boîtier (1) et dans l'espace d'outil (81), l'outil (4) est relativement sorti par rapport au capot de douche (8) de façon qu'entre la face arrière du disque (45) et le fond de capot (82) il y ait un espace pour la sortie de l'eau et la face frontale du disque (45) dotée de saillies de fraisage (46) est en position frontale de fraisage,
et **en ce que** dans la position de douche du capot de douche (8) avec le commutateur (7), le capot de douche (8) est sorti dans le boîtier (1) vers l'extérieur et dans l'espace d'outil (81) l'outil (4) est relativement rentré par rapport au capot de douche (8) de façon que la face arrière du disque (45) s'appuie sur le fond de capot (82) et la face frontale du disque (45) dotée de saillies de fraisage (46) est rentrée dans l'espace d'outil (81).
